# FASCICULE DE BREVET EUROPEEN

(11) **EP 2 554 113 B1**
(45) Date de publication et mention de la délivrance du brevet: **14.09.2016**
(21) Numéro de dépôt: 12190327.2
(22) Date de dépôt: 06.11.2008
(51) Int. Cl.: H02K 47/04, H02K 53/00, A61B 5/00, A61B 5/0488, A61B 8/00

(54) **Dispositif et methode de reperage non invasif d'une structure tel qu'un nerf**
Vorrichtung und Methode zur nicht-invasiven Lokalisierung einer Struktur, wie beispielsweise eines Nervs
Non-invasive device and method for locating a structure such as a nerve

(30) Priorité: 08.11.2007 FR 0758884
(43) Date de publication de la demande: 06.02.2013
(62) Demande divisionnaire de: 08853946.5
(73) Titulaire: Theraclion SA, 92240 Malakoff (FR)
(72) Inventeur: Lacoste, Francois, 94250 Gentilly (FR)
(74) Mandataire: Hepp Wenger Ryffel AG

(56) Documents cités:
- US-A1- 2005 240 126

## Description

La présente invention concerne le repérage non invasif de structures, tels que des nerfs, dans une zone d'un corps. Le repérage peut être une étape préalable à un traitement de tissus voisins de la structure.

Il est souvent nécessaire en médecine de repérer les nerfs, en particulier les nerfs moteurs, pour par exemple exciter un muscle ou encore éviter de détruire le nerf lors du traitement de tissus voisins.

Généralement on connaît la position approximative des nerfs par les atlas anatomiques, mais il peut être important de connaître leur position exacte dans une zone du corps, par exemple quand on veut effectuer des actes thérapeutiques dans son voisinage immédiat, sachant qu'on désire généralement le préserver. Le geste thérapeutique peut être une dissection ou consister à appliquer de l'énergie, par exemple ultrasonore, lumineuse (laser), radiofréquence ou encore micro-ondes ou radioactive. Connaissant sa position, on évitera de diriger le geste thérapeutique sur ce nerf ou juste à côté afin de le préserver.

Dans le cas par exemple du traitement des thyroïdes et des parathyroïdes, il est particulièrement important de préserver les nerfs laryngés récurrents, qui contrôlent les cordes vocales. Un autre exemple concerne les ablations de la prostate où il est important de préserver les nerfs érecteurs.

Il est aussi intéressant de repérer des nerfs sensitifs pour par exemple comprendre l'origine d'une douleur, ou les détruire afin de supprimer une douleur.

Le repérage précis des nerfs pose souvent problème, particulièrement lorsqu'il doit être effectué de façon non invasive, hors d'un contexte chirurgical. Les médecins utilisent l'imagerie (échographie, IRM, RX, scanner) pour repérer les structures anatomiques mais les nerfs ne se voient en général pas avec ces appareils en raison de leur petite taille ou d'un contraste insuffisant. Or de nombreuses méthodes thérapeutiques modernes sont " non invasives ", c'est-à-dire non chirurgicales ; le geste est alors uniquement guidé par l'imagerie.

En général, si l'acte thérapeutique consiste en une chirurgie, le chirurgien peut voir les nerfs grâce à une dissection prudente et soignée. En cas de doute, il peut aussi utiliser un Moniteur d'intégrité nerveuse tel que le
NIM-Response(R), Nerve Integrity Monitoring System, commercialisé par la société Xomed. Cet appareil comporte des électrodes que le chirurgien place à proximité des nerfs à repérer (par exemple les nerfs laryngés récurrents) et une sonde trachéale, qui est installée dans la trachée du patient pendant les traitements. Un courant d'excitation émis par les électrodes parcourt le tissu puis le nerf ; les mouvements des muscles ou l'excitation électrique qu'ils reçoivent sont captés par la sonde trachéale. D'autres stimulateurs sont commercialisés, tels que The Silverstein(TM) Facial Nerve Monitor/Stimulator, Model S8n de la société WR Médical Electronics Co. On peut aussi détecter le mouvement des cordes vocales par endoscopie.

Ces techniques permettent ainsi de détecter des altérations éventuelles de ces nerfs pendant le geste thérapeutique. Ceci a été décrit dans la demande EP 1 409 079.

Cependant lorsque le traitement est effectué à distance de la cible, par exemple par des ultrasons focalisés (HIFU) ou au moyen d'un laser interstitiel ou d'une aiguille radiofréquence (RF), on n'a pas d'accès direct au nerf et on cherche donc un dispositif et une méthode non invasifs de repérage pour localiser avec précision ces nerfs, ou plus généralement des structures. Pour atteindre ce but, la présente invention propose un dispositif de repérage non invasif d'une structure, telle qu'un nerf, dans une zone d'un corps ayant une surface externe, le dispositif comprenant un transducteur ultrasonore focalisé, avantageusement du type HIFU, adapté à produire un faisceau d'ultrasons à travers la surface externe dans la zone, le faisceau ayant un point focal capable d'être positionné sur la structure, des moyens de monitoring pour détecter une réponse de la structure lorsque soumise à une stimulation, et des moyens de stimulation pour stimuler la structure qui délivre une réponse détectée par les moyens de monitoring, les moyens de stimulation comprennent le transducteur ultrasonore capable de délivrer un faisceau d'ultrasons à ladite structure, pour la perturber, caractérisé en ce que le dispositif comprend un appareil d'imagerie couplé mécaniquement au transducteur ultrasonore de sorte que l'appareil d'imagerie suit le point focal du transducteur pour visualiser sur une image la position du point focal du faisceau d'ultrasons dans la zone de corps, des moyens de marquage pour marquer la position de la structure sur l'image produite par l'appareil d'imagerie. Avantageusement, les moyens de stimulation comprennent au moins une électrode capable de délivrer une impulsion électrique à ladite structure.

L'invention consiste donc à insonifier la région d'intérêt selon une méthode et un dispositif tels qu'ils sont définis dans les revendications 1 et 4, respectivement.

L'invention sera maintenant plus amplement décrite en référence aux dessins joints donnant à titre d'exemple non limitatif un mode de réalisation de l'invention.

Sur les figures :
La figure 1 est une vue schématique d'un dispositif de repérage selon l'invention,
La figure 2 est également une vue schématique montrant une partie du dispositif de repérage de la figure 1 en place sur un patient, et
Les figures 3 et 4 sont des organigrammes pour comprendre les différentes étapes de la méthode de repérage de l'invention.

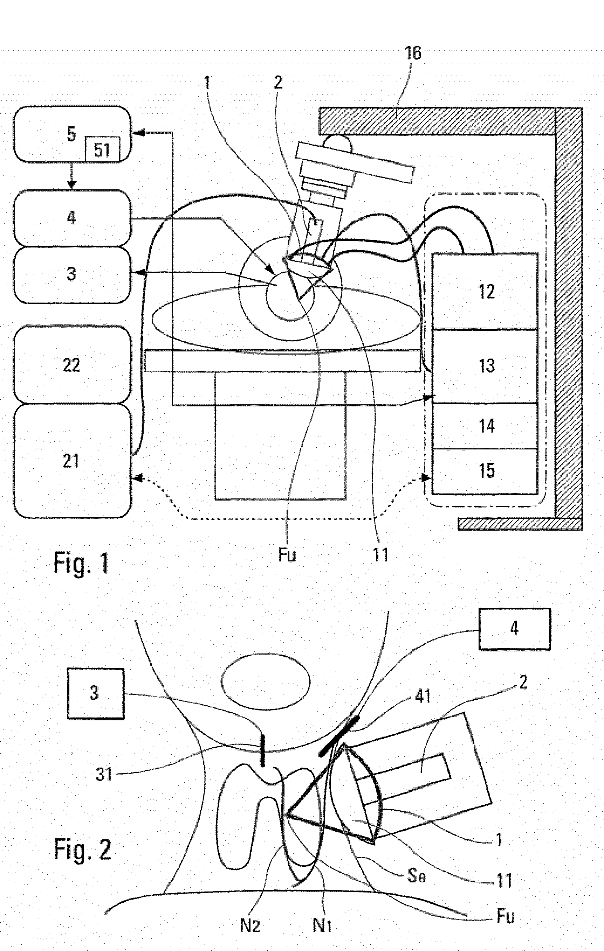

Pour des raisons de simplicité, il sera fait référence uniquement à un nerf dans la suite de la description comme exemple de structure cible à repérer à l'aide du dispositif et de la méthode de repérage non invasif de la présente invention. Bien entendu, d'autres types de structures que les nerfs peuvent être repérés grâce à la présente invention, que ces structures soient situées à l'intérieur d'un corps vivant ou d'un autre type de corps. II sera d'abord fait référence à la figure 1 pour décrire en détail les différents éléments constitutifs du dispositif de repérage de l'invention.

Le dispositif comprend tout d'abord une source d'émission acoustique 1 qui peut avantageusement être un transducteur ultrasonore adapté à produire un faisceau d'ultrasons Fu. De préférence, le transducteur ultrasonore est du type focalisé HIFU permettant de produire un faisceau focalisé d'ultrasons en un point focal précis. Le transducteur peut aussi être du type à barrettes. Le transducteur 1 , comme on peut le voir sur la figure 2, peut comprendre une chambre remplie d'un fluide de couplage à travers lequel se propagent les faisceaux d'ultrasons. La chambre peut par exemple être délimitée par un ballonnet souple destiné à venir en contact intime avec une surface externe Se d'une zone d'un corps où se situe le nerf à rechercher. En général, la surface externe Se est la peau du patient. Pour faire circuler le liquide de couplage à l'intérieur de la chambre 11 , il est en général prévu des moyens de circulation 12 qui permettent de réguler le débit et la température du fluide de couplage à l'intérieur de la chambre 11.

Le transducteur pour fonctionner a bien entendu besoin d'une alimentation de puissance 13 ainsi que d'une commande de déplacement 14 qui permet de déplacer et de localiser avec précision le transducteur par rapport au patient. Pour ce faire, le transducteur 1 est de préférence monté sur un bras articulé 16. Enfin, le transducteur est couplé à un ordinateur 15 qui permet de gérer tous les paramètres du transducteur, comme sa puissance, sa fréquence, sa durée d'impulsion, etc.

Le dispositif de repérage de l'invention comprend également des moyens d'imagerie qui peuvent par exemple se présenter sous la forme d'une sonde échographique 2 couplée à un échographe 21 et un écran de visualisation 22. La sonde 2 est couplée mécaniquement au transducteur 1 comme on peut le voir sur les figures 1 et 2. Plus précisément, la sonde 2 et le transducteur 1 sont solidaires l'un de l'autre de sorte que la sonde 2 suit le point focal des faisceaux d'ultrasons Fu. La zone d'intensité maximale du faisceau d'ultrasons Fu est toujours représentée sur l'image de l'écran 22. Pour cela l'échographe 21 peut être couplé à l'ordinateur 15 du transducteur comme on peut le voir sur la figure 1. A la place d'une imagerie échographique, on peut utiliser une imagerie IRM, RX ou un scanner.

Le dispositif de repérage de l'invention comprend également des moyens de monitoring 3 pour détecter une réponse du nerf lorsqu'il est soumis à une stimulation. La réponse du nerf peut être détectée directement au niveau du nerf sous la forme d'une impulsion électrique de réponse ou encore au niveau d'un muscle que le nerf commande. Dans ce dernier cas, la réponse du muscle peut se présenter sous la forme de l'activité électrique du muscle, d'une activité détectable visuellement ou encore d'un changement de pression ou une force. Ainsi, on peut utiliser en tant que moyens de monitoring des appareils du type endoscope, électromyographe, jauge de pression, etc. La jauge de pression peut par exemple être placée sur le muscle cricorayténoïde postérieur. A vrai dire, le type de moyen de monitoring n'est pas un élément critique pour la présente invention, il suffit qu'il puisse détecter une réponse du nerf soumis à une stimulation, que cette stimulation soit électrique, acoustique ou autre.

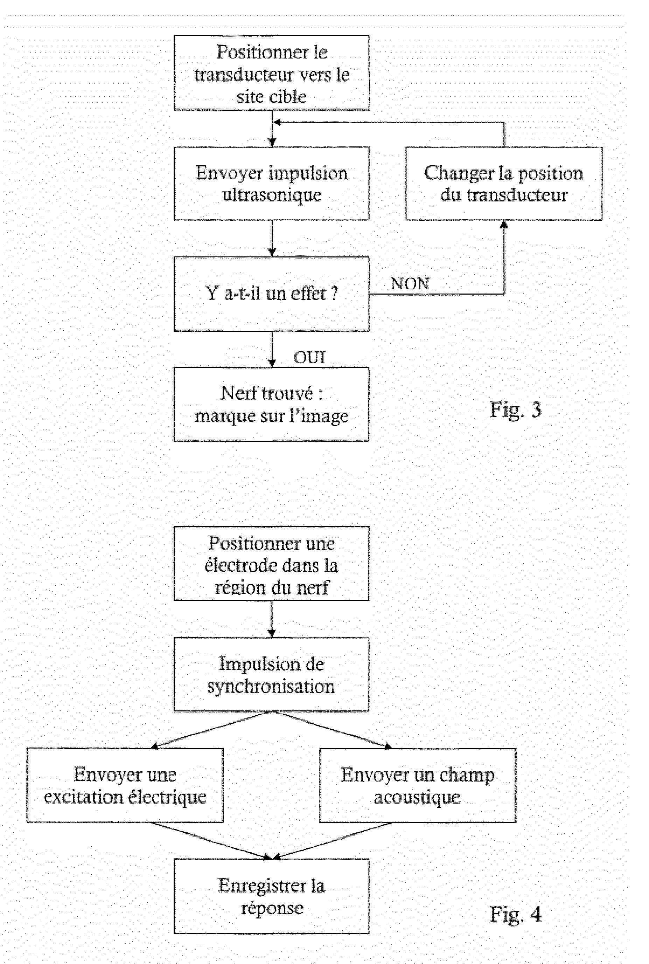

Le dispositif de repérage comprend également des moyens de stimulation acoustique. Sur la figure 1, le bloc 4 représente des moyens de stimulation électriques, ne faisant pas partie de l'invention, comprenant une électrode d'excitation ou de stimulation 41 destinée à être placée de manière à exciter le nerf recherché ou un autre nerf connecté au nerf recherché. Ceci est par exemple le cas sur la figure 2 sur laquelle on peut voir un nerf Ni, en l'occurrence le nerf vagal, et un nerf N2, en l'occurrence le nerf récurrent laryngé. L'électrode d'excitation reliée au stimulateur électrique 4 est placée de telle manière à pouvoir exciter le nerf vagal Ni qui va exciter le nerf récurrent laryngé N2. Le transducteur 1 avec sa sonde échographique associée 2 est appliqué contre la peau du cou de manière à insonifier les tissus du cou et ainsi envoyer les faisceaux d'ultrasons Fu sur le nerf récurrent laryngé N2. A la place de la stimulation électrique, on peut aussi stimuler le nerf à l'aide de faisceau d'ultrasons. On peut donc se servir du transducteur 1 pour stimuler le nerf. La réponse du nerf peut être surveillée par monitoring au niveau du muscle commandé par le nerf stimulé. II est déjà connu dans l'art antérieur qu'un faisceau acoustique suscite perturbe, diminue, augmente ou annule la transmission électrique à l'intérieur des nerfs. Cette capacité des faisceaux acoustiques est ici mise à contribution pour repérer la position d'un nerf. Le principe de l'invention repose sur le fait qu'une réponse perturbée ou induite du nerf est produite lorsque le faisceau d'ultrasons est positionné sur le nerf. Lorsque le nerf est stimulé à l'aide d'impulsions électriques générées par exemple par une technique transcutanée, tel que le Nerve Stimulator Multi Liner de la société TOENIES, il est possible de faire un monitoring de la réponse du nerf ou encore la réponse du muscle auquel le nerf est relié. La réponse détectée par le monitoring va être perturbée par le faisceau d'ultrasons issu du transducteur lorsque celui-ci est positionné sur le nerf. Le transducteur doit donc être déplacé dans la zone du corps où est situé le nerf jusqu'à ce qu'une perturbation de la réponse soit détectée sur les moyens de monitoring. Le transducteur est ainsi déplacé jusqu'à ce que l'on détecte ce changement dans la réponse du nerf. La figure 3 schématise de manière analytique les différentes étapes du procédé de repérage de l'invention. On positionne le transducteur vers la cible, on envoie l'impulsion ultrasonique, on observe s'il y a un effet sur la réponse du nerf. S'il n'y a aucun effet, on change la position du transducteur et on reprend la manipulation. S'il y a un effet, cela signifie que le faisceau est positionné sur le nerf et il faut alors marquer cette position sur l'image visualisée au niveau de l'écran de l'appareil d'imagerie.

Lorsque la stimulation est réalisée à l'aide du transducteur ultrasonore, on peut faire un monitoring de la réponse du nerf au niveau du muscle. Si le muscle réagit, cela signifie à nouveau que le faisceau d'ultrasons est positionné sur le nerf. La réaction du muscle peut être monitorée à l'aide d'un électromyographe, d'un endoscope ou encode d'un capteur de pression.

Il est également possible d'exciter le nerf par le cerveau même du patient. On peut par exemple demander au patient d'effectuer une action et constater si cette action peut toujours être effectuée pendant ou juste après l'insonification. Par exemple, si on veut repérer les nerfs laryngés récurrents, on demandera au patient d'émettre un son, par exemple monotonal et on enregistrera soit les modifications du son de la voix soit les modifications des caractéristiques de l'influx nerveux arrivant sur les muscles par les méthodes décrites ci-dessus.

Le faisceau d'ultrasons influx issu du transducteur 1 ne doit bien entendu pas être à même de détruire le nerf où les tissus environnants dans la zone de recherche. On ne dépassera pas des densités d'énergie (calculées comme le produit de l'intensité ultrasonore par la durée des impulsions) de 1000 watts par cm<2> x 1000 millisecondes. De préférence, on ne dépassera pas un io<eme> de cette valeur. On pourra aussi utiliser des puissances ou intensités ultrasonores fortes mais en impulsion unique (Single Puise), par exemple proche de celle utilisée pour la lithotritie extracorporelle des calculs rénaux : typiquement, la puissance du faisceau acoustique sera située entre 100 watts par cm<2> et 100 000 watts par cm<2> et la durée de l'impulsion sera située entre 1 microseconde et 1 milliiseconde, la pression maximale étant de 1 à 500 bars. Les fréquences ultrasonores seront de quelques MHz, entre 0,5 et 10 MHz, et plus précisément entre 2 et 4 MHz. Les impulsions ultrasonores peuvent être simples ou multiples, longues ou courtes.

Le dispositif de repérage comprend, ne faisant pas partie de l'invention, des moyens de synchronisation 5 couplés à la fois au transducteur 1 et aux moyens de stimulation 4 pour synchroniser le faisceau d'ultrasons Fu et l'impulsion électrique de manière à ce qu'ils parcourent le nerf en même temps. Cela permet d'optimiser l'effet du faisceau d'ultrasons sur la réponse électrique du nerf. La synchronisation peut prendre en compte les temps de propagation à la fois de l'influx nerveux et de l'onde ultrasonore dans le tissu afin que les impulsions soient globalement synchrones sur le site d'insonification, c'est-à-dire le nerf, et que l'effet de détection soit maximum. Dans la pratique, les moyens de synchronisation sont reliés à l'alimentation de puissance 13 du transducteur. Selon l'invention, les moyens de synchronisation comprennent également des moyens de décalage 51 adaptés à générer un décalage dans le temps entre le faisceau d'ultrasons et l'excitation électrique de sorte que le faisceau d'ultrasons arrive sur le nerf avant l'impulsion électrique. Un délai de 5 à 20 millisecondes est optimum pour diminuer ou augmenter la transmission de l'influx nerveux, de préférence 7 millisecondes. Ainsi, l'impulsion ultrasonore arrivera sur le nerf 7 millisecondes avant l'impulsion nerveuse, de manière à assurer que l'influx nerveux sera perturbé par l'impulsion ultrasonore. En pratique, les moyens de synchronisation délivrent une impulsion de synchronisation qui déclenche à la fois l'excitation électrique et le transducteur. La réponse du muscle peut alors être enregistrée. La figure 4 représente de manière schématique les différentes étapes de synchronisation mises en oeuvre par la méthode de repérage de l'invention. On commence par positionner l'électrode d'excitation sur la région du nerf, on émet ensuite l'impulsion de synchronisation, l'excitation électrique et le faisceau d'ultrasons sont envoyés et la réponse est détectée. Il est également possible à l'aide du dispositif de la méthode de repérage de l'invention de déterminer la profondeur du nerf, c'est-à-dire sa position dans la direction de propagation acoustique. En effet, le dispositif comprend des moyens de calcul permettant de déterminer la position en profondeur du nerf à partir du temps de vol acoustique du faisceau d'ultrasons et de la vitesse de propagation de l'influx nerveux.

Dans tous les cas, on excitera point par point la zone d'intérêt avec le faisceau d'ultrasons, en le déplaçant entre les tirs ou les séries de tirs. Lorsque la zone de concentration du faisceau (point focal) atteint le nerf, on obtient une réponse, tel que décrite plus haut. On notera alors la position du point focal, par exemple sur l'image échographique. On aura ainsi repéré le nerf à préserver sur les images échographiques de la région à traiter. II est à noter que le dispositif et la méthode de repérage de l'invention utilisent un transducteur ultrasonore ainsi qu'une sonde échographique qui peuvent ensuite être mis en oeuvre pour un traitement thérapeutique ultérieur des tissus avoisinants le nerf. Les seuls éléments additionnels sont alors les moyens de monitoring et les moyens de stimulation.

## Revendications

1. Méthode de repérage non invasive d'une structure (N2), telle qu'un nerf, dans une zone d'un corps ayant une surface externe (Se), comprenant
d'insonifier la structure (N2) à partir de la surface externe pour stimuler la structure (N2) avec un faisceau focalisé (Fu) en un point focal précis, produit d'un transducteur ultrasonore focalisé (1) du type HIFU,
de faire un monitoring de la réponse de la structure à la stimulation **caractérisée en ce qu'**elle comprend
- s'il y a un effet, de visualiser la position de l'insonification de la structure en marquant la position de la structure sur une image visualisée au niveau d'écran d'un appareil d'imagerie (2, 21, 22) couplé mécaniquement au transducteur ultrasonore focalisé (1) de sorte que l'appareil d'imagerie (2, 21, 22) et le transducteur ultrasonore focalisé (1) sont solidaires l'un de l'autre et l'appareil d'imagerie (2, 21, 22) suit le point focal des faisceaux d'ultrasons, ou ou
- s'il n'y a aucun effet, de changer la position du transducteur ultrasonore focalisé (1), **en ce que**
la profondeur de la structure (N2) est déterminée à partir du temps de vol acoustique du faisceau d'ultrasons (Fu) dans la zone jusqu'à ladite structure (N2) et la vitesse de propagation de la stimulation.

2. Méthode selon la revendication 1, **caractérisée en ce que** l'appareil d'imagerie (2, 21, 22) comprend une sonde échographique.

3. Méthode selon l'une quelconque des revendications 1 à 2, comprenant de positionner le transducteur ultrasonore focalisé (1) vers le site cible.

4. Dispositif de repérage non invasif d'une structure (N2), telle qu'un nerf, dans une zone d'un corps ayant une surface externe (Se), comprenant:
- un transducteur ultrasonore focalisé (1) du type
HIFU, adapté à produire un faisceau d'ultrasons (Fu) à travers la surface externe (Se) dans la zone jusqu'à ladite structure (N2) pour stimuler la structure (N2) qui délivre une réponse détectée par les moyens de monitoring (3),
- des moyens de monitoring (3) pour détecter une réponse de la structure lorsque soumise à une stimulation, et des moyens de stimulation (1),
**caractérisé en ce que** le dispositif comprend:
- un appareil d'imagerie (2, 21, 22) couplé mécaniquement au transducteur ultrasonore focalisé (1) de sorte que l'appareil d'imagerie (2, 21, 22) et le transducteur ultrasonore focalisé (1) sont solidaires l'un de l'autre et l'appareil d'imagerie (2, 21, 22) suit le point focal des faisceaux d'ultrasons pour visualiser la position du faisceau d'ultrasons (Fu) dans la zone de corps,
- des moyens des marquage pour marquer la position de la structure (N2) sur l'image produite par l'appareil d'imagerie s'il y a un effet et
- des moyens de changer la position du transducteur ultrasonore focalisé (1), et des moyens de calcul (15) pour déterminer la profondeur de la structure (N2) à partir du temps de vol acoustique du faisceau d'ultrasons (Fu) dans la zone jusqu'à ladite structure (N2) et la vitesse de propagation de la stimulation.

## Patentansprüche

1. Verfahren zum nichtinvasiven Orten einer Struktur (N2) wie etwa eines Nervs in einer Zone eines Körpers, die eine äußere Oberfläche (Se) besitzt, das Folgendes umfasst:
Beschallen der Struktur (N2) ausgehend von der äußeren Oberfläche, um die Struktur (N2) mit einem fokussierten Strahlenbündel (Fu) mit präzisem Brennpunkt, das von einem fokussierten Ultraschallwandler (1) des HIFU-Typs erzeugt wird, zu stimulieren,
Überwachen der Antwort der Struktur auf die Stimulation,
**dadurch gekennzeichnet, dass** es Folgendes umfasst:
- falls eine Wirkung auftritt, Anzeigen der Position der Beschallung der Struktur durch Markieren der Position der Struktur in einem Bild, das auf einem Schirm einer Bildgebungsvorrichtung (2, 21, 22) angezeigt wird, die mit dem fokussierten Ultraschallwandler (1) mechanisch gekoppelt ist, derart, dass die Bildgebungsvorrichtung (2, 21, 22) und der fokussierte Ultraschallwandler (1) fest miteinander verbunden sind und die Bildgebungsvorrichtung (2, 21, 22) dem Brennpunkt der Ultraschallbündel folgt, oder
- falls keine Wirkung auftritt, Ändern der
Position des fokussierten Ultraschallwandlers (1), und dass die Tiefe der Struktur (N2) anhand der Schallausbreitungszeit des Ultraschallbündels (Fu) in der Zone bis zu der Struktur (N2) und anhand der Ausbreitungsgeschwindigkeit der Stimulation bestimmt wird.

2. Verfahren nach Anspruch 1, **dadurch gekennzeichnet, dass** die Bildgebungsvorrichtung (2, 21, 22) eine Echographiesonde umfasst.

3. Verfahren nach einem der Ansprüche 1 bis 2, wobei das Verfahren das Positionieren des fokussierten Ultraschallwandlers (1) auf den Zielort umfasst.

4. Vorrichtung zum nichtinvasiven Orten einer Struktur (N2) wie etwa eines Nervs in einer Zone eines Körpers, die eine äußere Oberfläche (Se) besitzt, die Folgendes umfasst:
- einen fokussierten Ultraschallwandler (1) des HIFU-Typs, der dafür ausgelegt ist, ein Ultraschallstrahlenbündel (Fu) durch die äußere Oberfläche (Se) in der Zone bis zu der Struktur (N2) zu erzeugen, um die Struktur (N2) zu stimulieren, die eine Antwort liefert, die durch Überwachungsmittel (3) detektiert wird,
- Überwachungsmittel (3) zum Detektieren einer Antwort der Struktur, wenn sie einer Stimulation unterliegt, und Stimulationsmittel (1),
**dadurch gekennzeichnet, dass** die Vorrichtung Folgendes umfasst:
- eine Bildgebungsvorrichtung (2, 21, 22), die mit dem fokussierten Ultraschallwandler (1) mechanisch gekoppelt ist, derart, dass die Bildgebungsvorrichtung (2, 21, 22) und der fokussierte Ultraschallwandler (1) fest miteinander verbunden sind und die Bildgebungsvorrichtung (2, 21, 22) dem Brennpunkt der Ultraschallbündel folgt, um die Position des Ultraschallbündels (Fu) in der Körperzone anzuzeigen,
- Markierungsmittel, um die Position der Struktur (N2) in dem durch die Bildgebungsvorrichtung erzeugten Bild zu markieren, falls eine Wirkung auftritt, und
- Mittel zum Ändern der Position des fokussierten Ultraschallwandlers (1) und Berechnungsmittel (15), um die Tiefe der Struktur (N2) anhand der Schallausbreitungszeit des Ultraschallbündels (Fu) in der Zone bis zu der Struktur (N2) und anhand der Ausbreitungsgeschwindigkeit der Stimulation zu bestimmen.

## Claims

1. Non-invasive method for locating a structure (N2), such as a nerve, in a zone of a body having an outer surface (Se), comprising insonifying the structure (N2) from the outer surface to stimulate the structure (N2) with a beam (Fu) focussed at a precise focal point, produced by a focussed ultrasound transducer (1) of the HIFU type, monitoring the response of the structure to the stimulation, **characterized in that** it comprises
- if there is an effect, displaying the position of the insonification of the structure by marking the position of the structure on an image displayed on a screen of an imaging apparatus (2, 21, 22) mechanically coupled to the focussed ultrasound transducer (1) such that the imaging apparatus (2, 21, 22) and the focussed ultrasound transducer (1) are secured to one another and the imaging apparatus (2, 21, 22) tracks the focal point of the ultrasound beams, or
- if there is no effect, changing the position of the focussed ultrasound transducer (1), and **in that**
the depth of the structure (N2) is determined from the acoustic time of flight of the ultrasound beam (Fu) in the zone to said structure (N2) and the rate of propagation of the stimulation.

2. Method according to Claim 1, **characterized in that** the imaging apparatus (2, 21, 22) comprises an echographic probe.

3. Method according to either one of Claims 1 or 2, comprising positioning the focussed ultrasound transducer (1) towards the target site.

4. Non-invasive device for locating a structure (N2), such as a nerve, in a zone of a body having an outer surface (Se), comprising:
- a focussed ultrasound transducer (1) of the HIFU type, suitable for producing an ultrasound beam (Fu) through the outer surface (Se) in the zone to said structure (N2) to stimulate the structure (N2) which delivers a response detected by the monitoring means (3),
- monitoring means (3) for detecting a response of the structure when subjected to a stimulation, and stimulation means (1), **characterized in that** the device comprises:
- an imaging apparatus (2, 21, 22) mechanically coupled to the focussed ultrasound transducer (1) such that the imaging apparatus (2, 21, 22) and the focussed ultrasound transducer (1) are secured to one another and the imaging apparatus (2, 21, 22) tracks the focal point of the ultrasound beams to display the position of the ultrasound beam (Fu) in the zone of the body,
- marking means for marking the position of the structure (N2) on the image produced by the imaging apparatus if there is an effect and
- means for changing the position of the focussed ultrasound transducer (1), and
computation means (15) for determining the depth of the structure (N2) from the acoustic time of flight of the ultrasound beam (Fu) in the zone to said structure (N2) and the rate of propagation of the stimulation.
